# EUROPEAN PATENT SPECIFICATION

(11) **EP 0 701 544 B1**
(45) Date of publication and mention of the grant of the patent: **13.01.1999**
(21) Application number: 94905199.9
(22) Date of filing: 28.01.1994
(51) Int. Cl.: C07C 11/04, C07C 9/06, C07C 2/84

(54) **METHOD AND APPARATUS FOR FORMING ETHYLENE OR ETHANE AND ETHYLENE FROM METHANE**
VERFAHREN UND VORRICHTUNG ZUR HERSTELLUNG VON ETHYLEN ODER ETHAN UND ETHYLEN AUS METHAN
PROCEDE ET APPAREIL DE FORMATION D'ETHYLENE ET/OU D'ETHANE A PARTIR DE METHANE

(43) Date of publication of application: 20.03.1996
(73) Proprietor: Vayenas, Constantinos G., 263 31 Aroi, Patras (GR); Yentekakis, Ioannis V., 264 41 Patras (GR); Jiang, Yi, 264 43 Patras (GR)
(72) Inventor: Vayenas, Constantinos G., 263 31 Aroi, Patras (GR); Yentekakis, Ioannis V., 264 41 Patras (GR); Jiang, Yi, 264 43 Patras (GR)
(74) Representative: Argyriadis, Korinna
(86) International application number: GR9400001
(87) International publication number: WO9520556

(56) References cited:
- EP-A- 0 418 974
- SCIENCE vol. 262 , 8 October 1993 , LANCASTER, PA US pages 221 - 223 A. L. TONKOVICH ET AL 'Enhanced C2 Yields from Methane Oxidative Coupling by Means of a Separative Chemical Reactor' cited in the application

## Description

This invention relates to a method, apparatus and catalyst for forming C₂ hydrocarbons, i.e. ethylene or ethane and ethylene from methane or natural gas.

### Background of the invention

The oxidative dimerization or "coupling" of methane to C₂ hydrocarbons has been the object of numerous papers, patents and reviews during the last fifteen years (e.g. Keller and Bhasin 1982, Hinsen and Baerns 1983, Ito and Lunsford 1985, Lee and Oyama 1988, Jones et al 1986, Otsuka et al 1990, Eng and Stoukides 1991, Tsiakaras and Vayenas 1993, and references therein).

Several catalysts have been found which give selectivity to C₂ hydrocarbons, hereafter denoted by 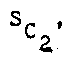 higher than 90% at low (<2%) CH₄ conversion, hereafter denoted by 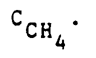 However it has been universally found (e.g. Keller and Bhasin 1982, Hinsen and Baerns 1983, Ito and Lunsford 1985, Lee and Oyama 1988, Jones et al 1986, Otsuka et al 1990, Eng and Stoukides 1991, Tsiakaras and Vayenas 1993, and references therein) that the selectivity 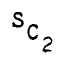 decreases rapidly with increasing conversion 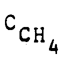 so that the C₂ hydrocarbon yield, hereafter denote by 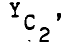 which equals the product of methane conversion 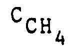 times the selectivity 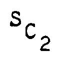 had been always found to be less than 30%.

In a recent paper Aris and coworkers (Tonkovich, Carr and Aris 1993) have shown that 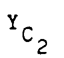 can be increased up to 50% by using a Sm₂O₃ catalyst in a simulated countercurrent moving bed chromatographic reactor (SCMBCR). The ethylene yield 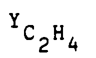 was up to 15% and the ethane yield 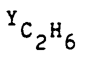 up to 30%.

The reason for the low selectivity 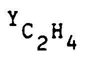 and 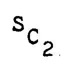 at high methane conversion 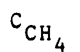 and thus the reason for the low measured 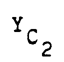 values is that the desired products ethylene or ethane and ethylene are far more reactive with oxygen than methane and therefore get easily converted to CO/CO₂ when their concentration becomes comparable to that of methane, i.e. for high methane conversions.

Consequently the observed improved C₂ yield ( 50%) in the case of the simulated countercurrent moving bed chromatographic reactor (SCMBCR, Tonkovich, Carr and Aris 1993) is due to the partial separation and removal of C₂ hydrocarbons from the CH₄. The same applies for the countercurrent moving bed chromatographic reactor (Tonkovich, Carr and Aris 1993).

The present invention provides the means for totally avoiding the problem of the high reactivity of C₂ hydrocarbons by trapping them quantitatively in a suitable adsorbent or absorbent material maintained at a lower temperature and thus permits obtaining, as shown in the examples, C₂ hydrocarbon yield 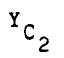 in excess of 80% and, more importantly, ethylene yield 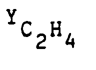 in excess of 75%.

### Brief description of the invention

This invention provides a means for converting methane or natural gas via partial oxidation to ethylene or ethane and ethylene and comprises a number n (n≥1) of units each consisting of (I) a catalyst or catalytic reactor (II) an oxygen feedthrough and (III) a suitable adsorbent or absorbent material maintained at a temperature significantly lower than that of the catalyst for continuously and quantitatively trapping ethane and ethylene. The n units are connected in series and any portion of the gas leaving each unit can be recirculated, if desirable, to any previous unit via a recirculation pump.

The catalyst of catalytic reactor may comprise catalyst particles in a fixed or fluidized bed or it may comprise catalytic material coated on the reactor walls.

The oxygen feedthrough is necessary in each unit to ensure that the methane to oxygen ratio in each catalyst unit can be controlled and maintained at desired levels. In general high methane to oxygen ratios favour high selectivity to C₂ hydrocarbons and to ethylene. Oxygen is supplied either via a gas feedthrough or electrochemically via a solid electrolyte cell (Vayenas et al 1990, 1992 and Tsiakaras and Vayenas 1993).

The adsorbent or absorbent material can be in the form of granules in a fixed or fluidized bed and forms a molecular sieve trap for ethane and ethylene. Two or more molecular sieve traps are used interchangeably in each unit so that one is trapping ethane and ethylene at a lower temperature (typically below 100°C) while the other is releasing it at a higher temperature. Alternatively the trapping material is continuously recirculated between the trapping zone which is maintained via cooling at a lower temperature (typically below 100°C) and a desorption zone where the trapped ethane and ethylene are released via heating.

### Brief description of the drawings

- Fig. 1: Shows schematically an apparatus suitable for carrying out the process of this invention. The feed consists of methane or a methane-oxygen mixture. Rⁱ (1≤i≤n) denotes a catalytic reactor, TR denotes a molecular sieve trap containing adsorbent or absorbent material.
- Fig. 2: Shows schematically another version of the apparatus shown in Fig. 1 with movable molecular sieve traps.
- Fig. 3: Shows schematically another version of the apparatus shown in Figures 1 and 2 with recirculating adsorbent or absorbent solid material.
- Fig. 4: Shows a fluidised bed recirculating solid apparatus which is also suitable for carrying out the process of this invention. The adsorbent or absorbent material is being continuously recirculated. The catalyst material is fixed on the reactor walls or is also recirculated continuously.
- Fig. 5: Shows schematically the version of the experimental apparatus shown in Fig. 1 used to obtain the results presented in the Examples and in subsequent figures; 6PV: six-port-valve; G.C.: gas chromatograph; T.C.: Thermocouple; MST: molecular sieve trap, 4PV: Four-port-valve, FCR: Fuel cell reactor, W: working electrode; C: counter electrode; R: reference electrode; P/G: potentiostatgalvanostat, 3WV: three-way-valves, R.P.: recirculation pump, B.F.: bubble flowmeter. Numbers and symbols are also explained in Example 1.
- Fig. 6: Shows typical results of the observed dependence of C₂ selectivity and C₂ yield on methane conversion for gas-phase supplied O₂ 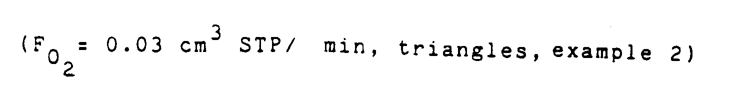 and electrochemically supplied O₂ (I= 7mA, squares, example 1); recirculation flow rate 220 cm³ STP/min, T= 835°C, initial methane partial pressure 20 kPa.
- Fig. 7: refers to Example 3 and shows typical results of the dependence of C₂ selectivity and C₂ yield on CH₄ conversion at various levels of electrochemical supply of oxygen I/4F, where I is the applied current.
- Fig. 8: refers to Example 3 and shows typical results of the dependence of the selectivity to ethylene 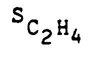 and to ethane 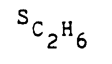 on the CH₄ conversion.
- Fig. 9: refers to Example 4 and to a pure Ag catalyst and depicts the dependence of C₂ selectivity and yield on CH₄ conversion.
- Fig. 10: refers to Example 5 and compares the performance of Y₂O₃-stabilized-ZrO₂ (triangles) and α-Al₂O₃ (circles) as supports for a 20%wt Sm₂O₃-80% Ag catalyst. The figure shows the effect of CH₄ conversion on C₂ selectivity and yield.
- Fig. 11: refers to Example 6 and shows the dependence of C₂ selectivity and yield and of ethylene yield on methane conversion for low values of applied current (I=5mA).

### Detailed description of specific embodiments

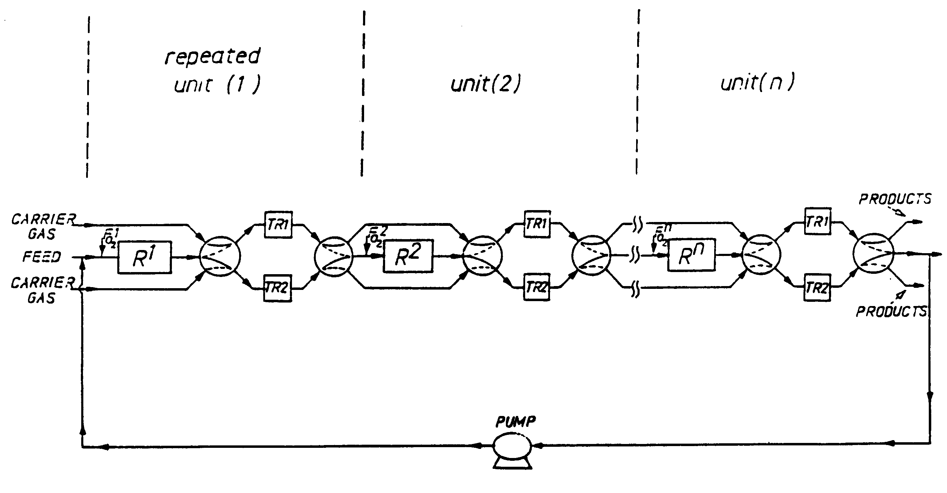

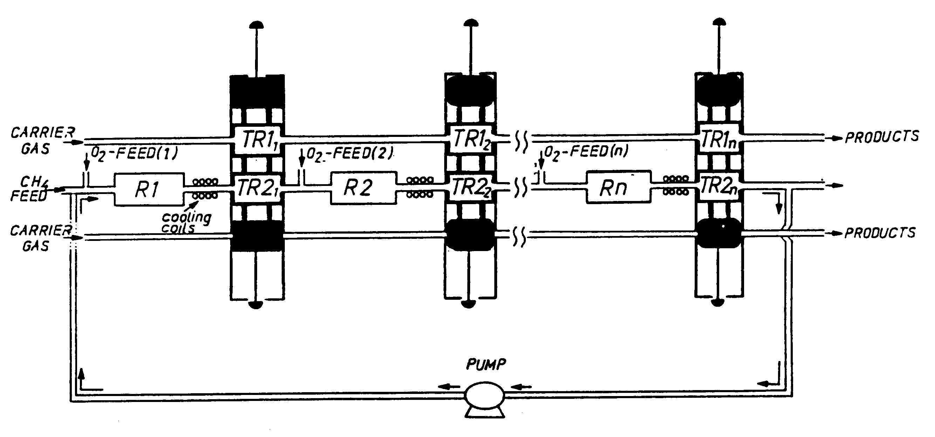

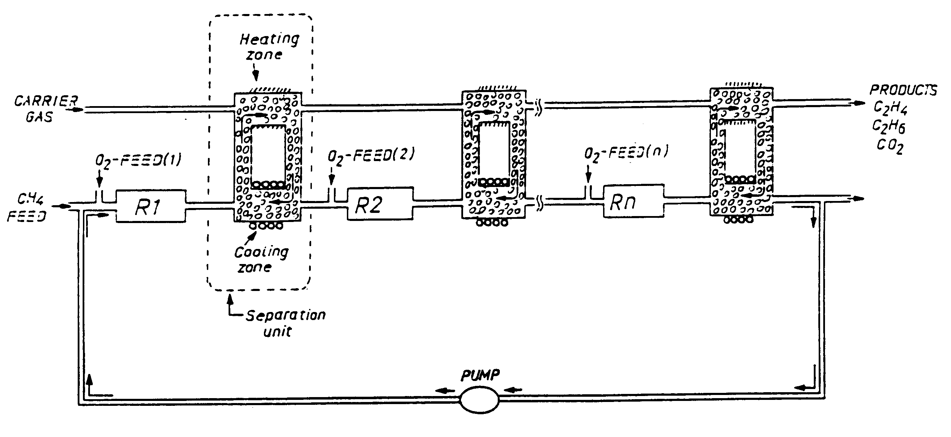

This invention provides a method and an apparatus for converting methane or natural gas to ethylene or to C₂ hydrocarbons, i.e. ethane and ethylene, which involves one or more successive contacts of a methane-oxygen-inert gas gaseous mixture with:
a) A catalyst or electrocatalyst suitable for producing C₂ hydrocarbons from methane.
b) A suitable adsorbent or absorbent material which can selectively trap ethane and ethylene, thus separating them from unreacted methane and oxygen. As shown in Figs. 1, 2 and 3 each basic unit of the apparatus contains, in addition to the catalytic or electrocatalytic reactor and the two, or more, interchangeable molecular sieve traps, and an oxygen feedthrough for addition of supplementary oxygen.

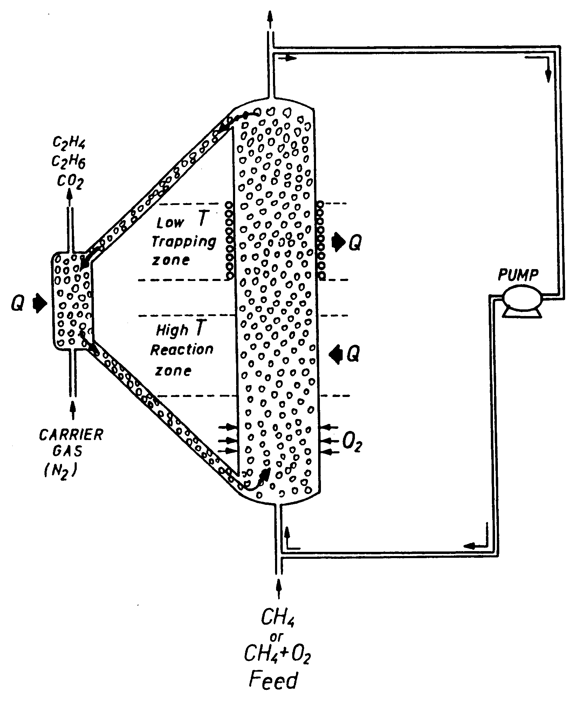

The catalyst can be in the form of pellets, or extrudates, in a fixed bed reactor or fine particles in a fluidized bed reactor (Fig. 4) or can be in the form of a film coated on the reactor walls. In this case the reactor walls can be fabricated from an O²-conducting solid electrolyte, such as yttria-stabilized-zirconia and oxygen can be supplied through the walls via application of a voltage between the catalyst film and a counter electrode film. In this case the voltage generated spontaneously between the catalyst film and the counter electrode, due to different oxygen activities, can be used to transport oxygen (Vayenas et al 1992). Mixed O²-electronic conductors can also be used.

The catalyst material can be any solid material which catalyzes the oxidative coupling of methane (OCM) to ethane and ethylene (e.g. Keller and Bhasin 1982, Hinsen and Baerns 1983, Ito and Lunsford 1985, Lee and Oyama 1988, Jones et al 1986, Otsuka et al 1990, Eng and Stoukides 1991, Tsiakaras and Vayenas 1993). We have found that Ag or Ag mixed with 1%CaO-doped-Sm₂O₃ is an excellent catalyst for the OCM reaction at high (larger than 2 to 1), CH₄ to O₂ ratios as these material, give 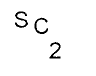 selectivity values above 95% for low CH₄ conversions.
Since these materials are also highly conductive, and therefore can, whenever desirable, be used in conjunction with Y₂O₃-stabilized-ZrO₂ as well, as electrochemical oxygen feedthroughs, these materials are particularly suitable for the present invention. The invention, however, refers to any material capable of catalyzing the OCM reaction.

The adsorbent or absorbent material in this invention can be any material capable of trapping selectively ethylene or ethane and ethylene without trapping methane and oxygen to any significant extent. We have found that molecular sieve 5A is very well suited for the present invention as it traps effectively ethane and ethylene at temperatures below 100°C and releases them reversibly at temperatures above 300°C. By using suitable adsorbent materials which trap ethylene more efficiently than ethane, such as e.g. molecular sieve 5A, it is possible to obtain very high ethylene yield values 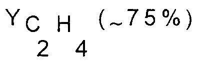 as shown on the Figures and Examples.

The present invention differs from all previous methods and apparatuses used to convert methane to ethylene or ethane and ethylene in that the gas mixture is brought continuously in repeated and successive contact with a catalyst (at a high (>500°C) temperature) and an adsorbent or absorbent material (at a low (<100°C) temperature) with intermediate continuous addition of oxygen so that the desired product ethylene or the desired products ethane and ethylene are trapped quantitatively in the adsorbent or absorbent material and thus are not allowed to further react with oxygen and form carbon monoxide and carbon dioxide.

The main differences of the present invention from the countercurrent moving bed chromatographic reactor (CMBCR) or Aris and coworkers (Tonkovich, Carr and Aris 1993 and references therein) are the following:
I. In the present invention the catalyst and the adsorbent are not mixed as in the CMBCR and the adsorbent always operates at temperatures much lower (typically by 600°C) than that of the catalyst, which again does not apply to the CMBCR.
II. In the present invention, with the exception of Fig. 4 and claims 7, 8, 9 and 10 discussed at the end of this paragraph, the catalyst and the adsorbent are at fixed positions, contrary to the CMBCR where they move. Figure 4 and claims 7, 8, 9 and 10 refer to a recirculating solid fluidized bed reactor which is again totally different from the CMBCR.
III. In the present invention the feed gas (methane or methane plus oxygen) is supplied at one end of the reactor and not in the middle of the reactor as in the CMBCR. The present invention also involves side feed of oxygen along the reactor length which is not the case with the CMBCR.
IV. The present invention, with the exception of Figure 4 and claims 7, 8, 9, 10 discussed above, consists of discrete units and not of a continuous moving bed.
V. In the present invention the gases are continuously recycled.

The main differences of the present invention from the simulated countercurrent moving bed chromatographic reactor (SCMBCR) of Aris and coworkers (Tonkovich, Carr and Aris 1993) are the following:
I. In the present invention the gas feed is constant and continuous and not switched between various units and interrupted as in the case of the SCMBCR.
II. All catalyst beds are continuously in contact with reacting gas and therefore are in continuous operation, contrary to the SCMBCR.
III. Product removal is carried out by raising the adsorbent temperature and thus no large volumes of carrier gas are needed.

As a result of all the above main differences the present invention has led already to 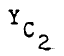 values above 80% and 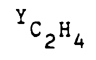 values above 75% vs 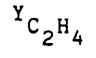 values of 50% and 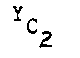 values of 15% for the SCMBCR and the CMBCR.

The following working Examples provides a more detailed description of the invention.

### Example 1

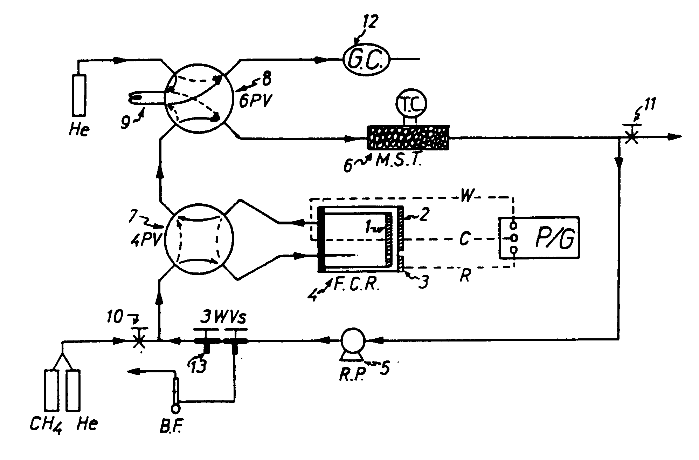

A porous catalyst film of total mass 150 mg consisting of 20wt% Sm₂O₃ doped with 1 wt% CaO and of 80 wt% Ag was deposited on the inside wall of an 8 mol% Y₂O₃-stabilized-ZrO₂ (YSZ) tube closed flat at one end as shown on Figure 5. Refering to Figure 5 the catalyst film 1 had a superficial area of 10 cm². Two Ag films (denoted 2 and 3) were deposited on the outside walls of the YSZ tube and acted as counter and reference electrodes respectively. The YSZ tube together with the Ag-Sm₂O₃-CaO catalyst thus formed the fuel-cell type catalytic reactor 4.

A recirculation pump 5 was used to recirculate the gas feed mixture comprising initially 20 mol% CH₄ in He between the YSZ reactor and the absorbent molecular sieve trap 6 containing 5 gr of molecular sieve 5A. In addition to the catalytic reactor, the molecular sieve trap and the recirculation pump, the recirculation loop also comprised a 4-port-valve 7 and a 6-port-valve 8 with a gas-sampling-loop 9. By appropriate setting the needle-valves 10 and 11 the apparatus could be operated either as a continuous flow reactor with gas recycle or as a batch reactor with recirculation. In this example the batch mode of operation with recirculation was chosen. The oxygen was supplied electrochemically by applying a constant current I=7mA via a galvanostat-potentiostat between the catalyst film 1 and the counter electrode 2. Thus oxygen is transported from the ambient air to the catalyst at a rate I/4F, where F is Faraday's constant, equal to 1.8·10⁻⁸ mol O₂/s. The Ag-Sm₂O₃-CaO catalyst films is maintained at T=835°C and now also acts as an electrocatalyst (Vayenas et al 1992, Tsiakaras and Vayenas 1993) and catalyzes the partial oxidation of CH₄ to ethane and ethylene which are being continuously trapped in the molecular sieve trap 6 which is maintained at T=30°C. The total volume of the recirculation loop is 50 cm³.

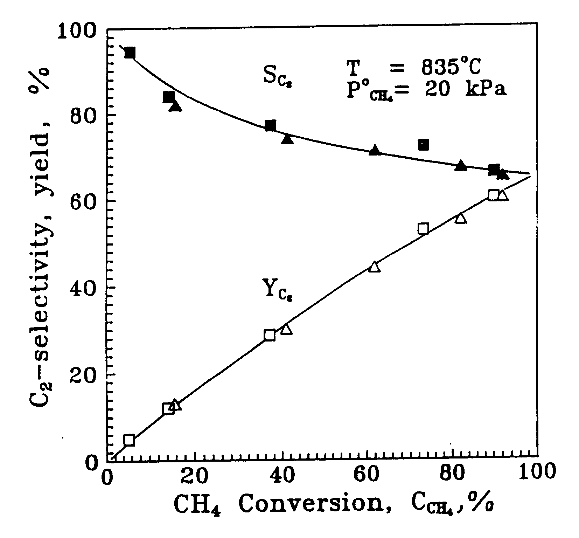

After operating the unit for a time t typically 5-300 min, the four-port-valve 7 is used so that the reactor is isolated from the loop and the temperature of the molecular sieve trap is raised to T=400°C thus causing desorption of the adsorbed ethane and ethylene. The composition of the gas present in the loop is then determined via the gas chromatograph 12 and the methane conversion 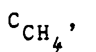 the product selectivities 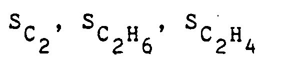 and the yields 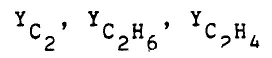 are then computed from the measured gas composition. The carbon mass closure is then also checked between the initial and final composition. By repeating the above experiment for various operating times t one varies the CH₄ conversion and thus obtains the results shown on Figure 6 (squares). Increasing methane conversion from zero to 95% causes a decrease in C₂ selectivity from 95% to 65% and an increase in C₂ yield from zero to 60%.

### Example 2

The same apparatus, catalyst and experimental procedure described in Example 1 is used but in the present case oxygen is not introduced via the YSZ solid electrolyte but from the gas phase via the needle valve 13 (Figure 5) at a flow rate of 3·10⁻² cm³ STP/min or equivalently 2.2·10⁻⁸ mol O₂/s, i.e. very similar to that used in Example 1. The results are shown on Figure 6 (triangles) and are almost identical to those obtained in Example 1. Maximum C₂ yield is again 60%.

### Example 3

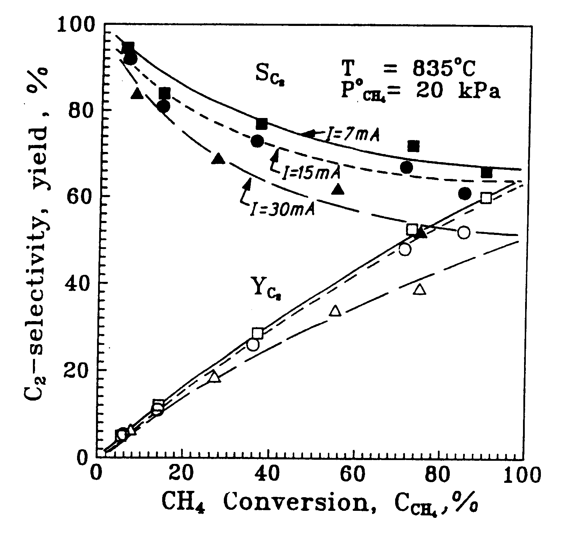

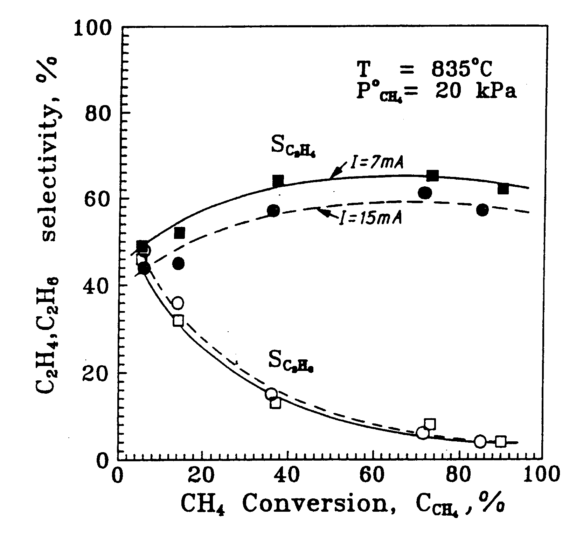

The same apparatus, catalyst and experimental procedure described in Example 1 was used and the effect of applied constant current I was investigated. As shown on Fig. 7 increasing I, i.e. increasing the rate I/4F of oxygen supply causes a small decrease in C₂ selectivity and yield. This is also shown on Figure 8 which shows additionally the variation in the selectivity to ethylene 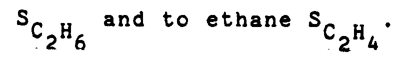 Increasing conversion of CH₄ via increasing operation time causes a decrease in 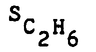 because ethylene is well known to form via oxidative dehydrogenation of C₂H₆ which is the primary product of the OCM reaction. It is clear from this Figure that the C₂H₆ formed in the reactor is not trapped 100% in the molecular sieve trap but some of it recirculates and forms C₂H₄. The results shown on Fig. 8 for high CH₄ conversions are of significant practical importance, since C₂H₄ is more valuable than C₂H₆.

### Example 4

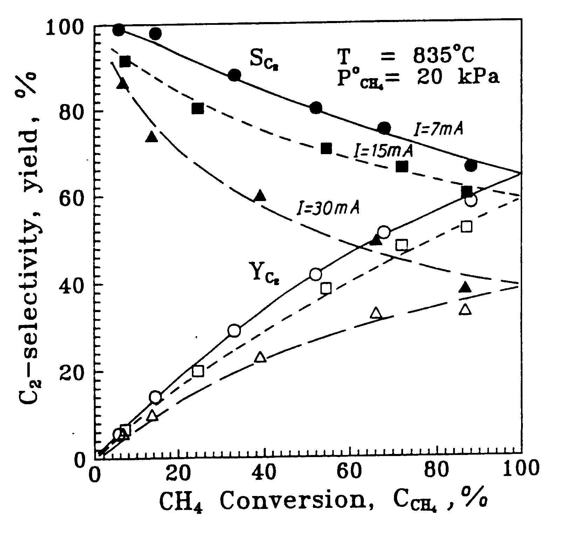

The same apparatus and experimental procedure described in Example 1 is used but in this case the catalyst is pure Ag (mass 129 mg, apparent surface area 18 cm²) deposited on the YSZ solid electrolyte. The results are shown on Figure 9 for three different values of the applied current I. Maximum C₂ yield is 59%. This shows in conjunction with example 1 that the addition of Sm₂O₃ (1% CaO) has only a small beneficial effect in the Ag catalyst performance.

### Example 5

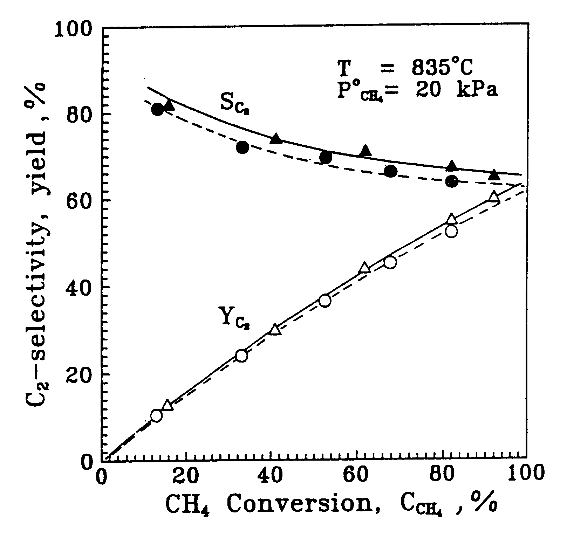

The same apparatus, catalyst and experimental procedure of Example 2 is used and two different catalyst supports are examined under conditions of gas-phase oxygen addition: Yttria-stabilized-zirconia (YSZ) as in Example 2 (triangles Figure 10) and α-Al₂O₃ (circles, Figure 10). Maximum C₂ yield is again near 60% and the catalyst support plays only a minor role in catalyst performance with YSZ being slightly better than α-Al₂O₃.

### Example 6

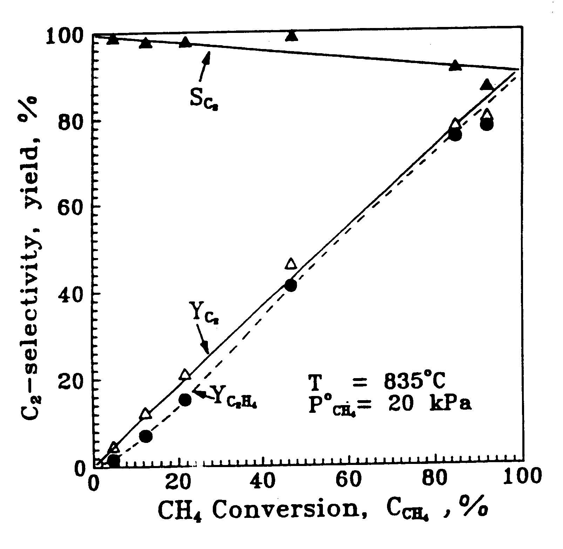

The same apparatus and experimental procedure described in Example 1 is used but in this case, as in example 4, the catalyst is pure Ag (mass 129 mg, apparent surface area 18 cm²) deposited on the YSZ solid electrolyte. In the present case a small current is applied (I=5mA). The molecular sieve trap contains 5 gr of molecular sieve 5A. Figure 11 shows the observed dependence of C₂ selectivity 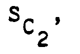 C₂ yield 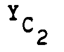 and ethylene yield 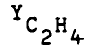 on methane conversion; 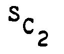 decreases slightly from 97% to 88% as the methane conversion increases from zero to 93% correspondingly. The C₂ yield increases from zero to 81% while the ethylene yield increases from zero to 78%. These are by far the highest C₂ and ethylene yields reported in the open or patent literature for the OCM reaction.

### Literature cited

Keller, G.E. and Bhasin, M.M., J.Catal. 73, 9 (1982)
Hinsen, W. and Baerns, M., Chem.Z. 107, 223 (1983)
Ito, T. and Lunsford, J.H., J.Phys.Chem. 87, 301 (1983)
Jones, C.A., Leonard, J.J. and Sofranko, J.A., U.S. Patent 4,567,307 (1986)
Lee, J.S. and Oyama, S.T. Catal.Rev.-Sci.Eng. 30(2), 249 (1988)
Otsuka, K., Suga, K. and Yamanaka, I., Catal.Today 6, 587 (1990)
Eng, D. and Stoukides, M., Catal.Rev.-Sci.Eng. 33, 375 (1991)
Tonkovich, A.L., Carr, R.W. and Aris, R., Science 262, 221 (1993) and references therein.
Vayenas, C.G., Bebelis, S. and Ladas, S., Nature (London) 343, 625 (1990)
Vayenas, C.G., Bebelis, S., Yentekakis, I.V. and Lintz, H.-G. Catalysis Today 11(3), 303 (1992) and references therein.
Tsiakaras, P. and Vayenas, C.G., J.Catal. 144, 333 (1993)

## Claims

1. Process for converting methane to ethylene or to ethane and ethylene which comprises successive and repeated contact of a methane-containing and oxygen-containing feed gas with a catalyst and with an adsorbent or absorbent material, said catalyst being capable of promoting the partial oxidation of methane to ethane and ethylene at temperatures above 500°C, said adsorbent or absorbent material maintained at a temperature typically below 100°C and being capable of adsorbing or absorbing ethylene or ethane and ethylene and subsequently releasing them at a higher temperature.

2. The process of claim 1 where the feed gas is a methane-containing gas and oxygen is added to said gas via a gas feedthrough between each contact with said catalyst and said adsorbent or absorbent material.

3. The process of claim 1 which comprises continuous passing of a methane-containing feed gas incontact with a series of catalysts or catalyst beds capable of promoting the conversion of methane to ethane and ethylene at temperatures above 500°C, each gas contact with one of said catalysts or catalyst beds followed by gas contact with an adsorbent or absorbent material contained in a different chamber capable of adsorbing or absorbing ethylene or ethane and ethylene at temperatures below 100°C.

4. The process of claim 3 where oxygen is added to said methane-containing gas between each contact with said adsorbent or absorbent material and said catalyst.

5. The process of any ones of claims 1 to 4 wherein said catalyst is supported on a solid electrolyte capable of transporting oxygen.

6. The process of any ones of claims 1 to 5 wherein part of the methane-containing gas is continuously recirculated for multiple contact with said catalyst and said adsorbent or absorbent material.

7. The process of claim 1 which comprises contact of a methane-containing and oxygen-containing feed gas with a catalyst and an adsorbent or absorbent material in a fluidized bed reactor with solid recirculation, said catalyst being capable of promoting the oxidative coupling of methane to ethane and ethylene at temperatures above 500°C, said adsorbent or absorbent material being capable of adsorbing or absorbing ethane and ethylene at temperatures below 100°C.

8. The process of claim 7 where part of said feed-gas is continuously recirculated for multiple contact with said catalyst and said adsorbent or absorbent material.

9. The process of any ones of claims 7 and 8 where said catalyst is supported on the reactor walls and said adsorbent material is recirculating between a low temperature and a high temperature zone.

10. The process of any ones of claims 7 and 8 where said catalyst and said adsorbent or absorbent material form composite solid particles.

11. The process of any one any one of claims 1 to 10 where ethane and ethylene are released from said adsorbent or absorbent material via heating.

12. The process of any one of claims 1 to 11 where said catalysts comprise a silver-containing silver-metal oxide composition.

## Patentansprüche

1. Verfahren zur Umwandlung vom Methan in Ethylen oder in Ethan und Ethylen, welches den succesiven und wiederholten Kontakt eines methanhaltigen und sauerstoffhaltigen eingespeisten Gases mit einem Katalysator und einem adsorbenten oder absorbenten Material einschließt, der erwähnte Katalysator in der Lage ist die teilweise Oxidation von Methan in Ethan und Ethylen bei Temperaturen über 500°C zu fördern, das erwähnte adsorbente oder absorbente Material bei einer Temperatur typisch unter 100° C gehalten wird und in der Lage ist Ethylen oder Ethan und Ethylen zu adsorbieren oder zu absorbieren und darauffolgend diese bei einer höheren Temperatur abzugeben.

2. Das Verfahren von Anspruch 1 wobei das eingespeiste Gas ein methanhaltiges Gas ist und Sauerstoff diesem Gas zugefügt wird durch eine Intergaszufuhr zwischen jedem Kontakt mit dem erwähnten Katalysator und dem erwähnten adsorbenten oder absorbenten Material.

3. Das Verfahren von Anspruch 1, welches den ständigen Durchfluß von methanhaltigem eingespeistem Gas in Kontakt mit einer Reihe von Katalysatoren oder Katalysatorbetten einschließt, die in der Lage sind die Umwandlung von Methan in Ethan und Ethylen bei Temperaturen über 500° C zu fördern, jedem Kontakt des Gases mit einem der erwähnten Katalysatoren oder Katalysatorbetten folgt ein Kontakt des Gases mit einem adsorbenten oder absorbenten Material, welches sich in einer unterschiedlichen Kammer befindet und in der Lage ist Ethylen oder Ethan und Ethylen bei Temperaturen unter 100° C zu adsorbieren oder zu absorbieren.

4. Das Verfahren von Anspruch 3 wobei dem erwähnten methanhaltigen Gas zwischen jedem Kontakt mit dem erwähnten adsorbenten oder absorbenten Material und dem erwähnten Katalysator Sauerstoff zugefügt wird.

5. Das Verfahren eines jeden der Ansprüche 1 bis 4 wobei der erwähnte Katalysator von einem festen Elektrolyten getragen wird, der in der Lage ist Sauerstoff zu transportieren.

6. Das Verfahren eines jeden der Ansprüche 1 bis 5 wobei ein Teil des methanhaltigen Gases ständig rezirkuliert zum multiplen Kontakt mit dem erwähnten Katalysator und dem erwähnten adsorbenten oder absorbenten Material.

7. Das Verfahren von Anspruch 1 welches den Kontakt eines methanhaltigen und sauerstoffhaltigen eingespeisten Gases mit einem Katalysator und einem adsorbenten oder absorbenten Material in einem Fließbettreaktor mit Rezirkulation fester Partikel einschließt, der erwähnte Katalysator in der Lage ist die oxidative Umwandlung von Methan in Ethan und Ethylen bei Temperaturen über 500° C zu fördern, das erwähnte adsorbente oder absorbente Material in der Lage ist Ethan und Ethylen bei Temperaturen unter 100° C zu adsorbieren oder zu absorbieren.

8. Das Verfahren von Anspruch 7 wobei ein Teil des erwähnten eingespeisten Gases ständig rezirkuliert zum multiplen Kontakt mit dem erwähnten Katalysator und dem erwähnten adsorbenten oder absorbenten Material.

9. Das Verfahren eines jeden der Ansprüche 7 und 8 wobei der erwähnte Katalysator auf den Reaktorwänden aufgetragen ist und das erwähnte adsorbente Material zwischen einer Zone mit niedriger und einer Zone mit hoher Temperatur rezirkuliert.

10. Das Verfahren eines jeden der Ansprüche 7 und 8 wobei der erwähnte Katalysator und das erwähnte adsorbente oder absorbente Material komposite feste Partikel formen.

11. Das Verfahren eines jeden der Ansprüche 1 bis 10 wobei Ethan und Ethylen vom erwähnten adsorbenten oder absorbenten Material durch Erhitzung abgegeben werden.

12. Das Verfahren eines jeden der Ansprüche 1 bis 11 wobei die erwähnten Katalysatoren aus einer silberhaltigen Silbermetalloxidkomposition bestehen.

## Revendications

1. Un procédé pour convertir du méthane à éthylène ou a éthane et éthylène, qui comprend le contact successif et répété d' un gaz d'alimentation, qui contient du méthane et de l' oxygène, avec un catalyseur et avec un matériel adsorbant ou absorbant, ledit catalyseur est capable de promouvoir l' oxydation partielle du méthane à éthane et éthylène aux températures plus hautes de 500°C, ledit matériel adsorbant ou absorbant est maintenu à une température typiquement plus basse de 100°C et est capable d' adsorber ou absorber l' éthylène ou l' éthane et l' éthylène et ensuite les libérer à une température plus haute.

2. Le procédé de la revendication 1, où le gaz d'alimentation est un gaz qui contient du méthane et on ajoute de l' oxygène audit gaz, par un réservoir d'alimentation, entre chaque contact avec ledit catalyseur et ledit matériel adsorbant ou absorbant.

3. Le procédé de la revendication 1, qui comprend le passage continuel d' un gaz d'alimentation qui contient du méthane, au contact avec une série des catalyseurs ou des couches de catalyseur qui sont capables de promouvoir la conversion du méthane à éthane et éthylène aux températures plus hautes de 500°C, chaque contact du gaz avec un desdits catalyseurs ou couches de catalyseur est suivi par le contact du gaz avec un matériel adsorbant ou absorbant, qui est contenu dans une chambre differente, qui est capable d' adsorber ou absorber l' éthylène ou l' éthane et l' éthylène aux températures plus basses de 100°C.

4. Le procédé de la revendication 3, où on ajoute de l' oxygène audit gaz, qui contient du méthane, entre chaque contact avec ledit matériel adsorbant ou absorbant et ledit catalyseur.

5. Le procédé d' une des revendications 1 à 4, où ledit catalyseur est supporté sur un électrolyte solide qui est capable de transporter l'oxygène.

6. Le procédé d' une des revendications 1 à 5, où une part du gaz qui contient du méthane est continuellement recirculé pour le contact multiple avec ledit catalyseur et ledit matériel adsorbant ou absorbant.

7. Le procédé de la revendication 1, qui comprend le contact d' un gaz d'alimentation qui contient du méthane et de l' oxygène avec un catalyseur et avec un matériel adsorbant ou absorbant dans un réacteur de couche fluidifiée avec recirculation solide, ledit catalyseur est capable de promouvoir le couplage oxydatif du méthane à éthane et éthylène aux températures plus hautes de 500°C, ledit matériel adsorbant ou absorbant est capable d'adsorber ou absorber l' éthane et l' éthylène aux températures plus basses de 100°C.

8. Le procédé de la revendication 7, où une part dudit gaz d'alimentation est continuellement recirculé pour le multiple contact avec ledit catalyseur et ledit matériel adsorbant ou absorbant.

9. Le procédé d' une des revendications 7 et 8, où ledit catalyseur est supporté aux parois du réacteur et ledit matériel adsorbant est recirculé entre une zone de température basse et une zone de température haute.

10. Le procédé d' une des revendications 7 et 8, où ledit catalyseur et ledit matériel adsorbant ou absorbant forment des particules solides composites.

11. Le procédé d' une des revendications 1 à 10, où l' éthane et l' éthylène sont libérer dudit matériel adsorbant ou absorbant par chauffage.

12. Le procédé d' une des revendications 1 à 11, où lesdit catalyseurs comprennent une composition d' oxyde du métal-argent contenant d'argent.
